# EUROPÄISCHE PATENTSCHRIFT

(11) **EP 4 122 387 B1**
(45) Veröffentlichungstag und Bekanntmachung des Hinweises auf die Patenterteilung: **18.12.2024**
(21) Anmeldenummer: 21187024.1
(22) Anmeldetag: 21.07.2021
(51) Int. Cl.: A61B 5/00, G01R 33/28, A61B 5/055

(54) **MAGNETRESONANZVORRICHTUNG MIT EINER PATIENTENDARSTELLUNGSEINHEIT**
MAGNETIC RESONANCE DEVICE COMPRISING A PATIENT REPRESENTATION UNIT
APPAREIL À RÉSONANCE MAGNÉTIQUE DOTÉ D'UNITÉ DE REPRÉSENTATION DU PATIENT

(43) Veröffentlichungstag der Anmeldung: 25.01.2023
(73) Patentinhaber: Siemens Healthineers AG, 91301 Forchheim (DE)
(72) Erfinder: Bollenbeck, Jan, 91330 Eggolsheim (DE)
(74) Vertreter: Siemens Healthineers Patent Attorneys

(56) Entgegenhaltungen:
- EP-A1- 3 581 109
- EP-A1- 3 660 530
- WO-A1-2016/068420
- WO-A1-2018/049196
- JP-B2- 6 656 881
- US-A1- 2006 079 763
- KIM DO YOON ET AL: "Stretchable and reflective displays: materials, technologies and strategies", NANO CONVERGENCE, vol. 6, no. 1, 20 June 2019 (2019-06-20), internet, XP055851163, Retrieved from the Internet <URL:https://nanoconvergencejournal.springeropen.com/track/pdf/10.1186/s40580-019-0190-5.pdf> [retrieved on 20211014], DOI: 10.1186/s40580-019-0190-5

## Beschreibung

Die vorliegende Erfindung betrifft eine Magnetresonanzvorrichtung mit einer Scannereinheit, einem von der Scannereinheit zumindest teilweise umgebenen Patientenaufnahmebereich und einer innerhalb des Patientenaufnahmebereichs angeordneten Patientendarstellungseinheit.

Magnetresonanzuntersuchungen an Patienten weisen eine relativ lange Untersuchungszeit auf. Während dieser Untersuchungszeit befindet sich der Patient, insbesondere der zu untersuchende Bereich des Patienten, innerhalb eines Patientenaufnahmebereichs der Magnetresonanzvorrichtung. Von daher besteht häufig die Notwendigkeit, dem Patienten Informationen und/oder Anweisungen während der Dauer der Magnetresonanzuntersuchung zu übermitteln. Aufgrund der zum Teil sehr lauten Betriebsgeräusche einer Magnetresonanzvorrichtung steht dem Patienten während der Magnetresonanzuntersuchung ein Gehörschutz zur Verfügung. Um dennoch eine Audiokommunikation des Patienten mit dem medizinischen Bedienpersonal während der Dauer der Magnetresonanzuntersuchung zu ermöglichen, ist es zudem bekannt, dass der Patient zusätzlich einen raumeinnehmenden Kopfhörer aufsetzt.

Jedoch ist eine Audiokommunikation für Patienten, die ein vermindertes Hörvermögen aufweisen, zusätzlich erschwert oder nicht möglich. Es ist daher wünschenswehrt, dass zusätzlich ein optischer und/oder visueller Kommunikationsweg für Patienten zur Verfügung steht. Ein Einbau eines Standard-LED-Displays oder eines LCDs (Liquid Crystal Display) in den während der Untersuchung vom Patienten einsehbaren Bereich, insbesondere innerhalb des Patientenaufnahmebereichs, gestaltet sich jedoch schwierig, da diese Displays starr sind und sich nicht an die Krümmung einer den Patientenaufnahmebereich umgebenden Umhausung anpassen lassen. Des Weiteren erzeugen derartige Displays elektromagnetische Störungen, die potenziell zu Bildartefakten in den erfassten Bilddaten führen können. Außerdem weisen derartige Displays einen hohen Leistungsumsatz auf.

Um eine visuelle Information in den Patientenaufnahmebereich für den Patienten zur Verfügung zu stellen, ist es bisher bekannt, dass eine Information an eine Wand projiziert wird und über eines Spiegelsystems in den Patientenaufnahmebereich zur Darstellung für den Patienten projiziert wird. Derartige Anordnungen mit einem Spiegelsystem beanspruchen jedoch sehr viel Platz innerhalb des Patientenaufnahmebereichs und engen somit den für den Patienten zur Verfügung stehenden Platz und/oder Raum ein. Ein derartiges Spiegelsystem ist in US 10241385 B2 offenbart.

WO 2018/049196 A1 offenbart ein Verfahren zum Anzeigen von Bilddaten eines Patienten auf einer tragbaren oder handgeführten Anzeigevorrichtung.

WO 2016/068420 A1 beschreibt ein medizinische Bildgebungsgerät, das einen Tisch, auf dem ein Subjekt liegt und der das Subjekt trägt, eine Gantry, die einen Innenraum umfasst, aufweist. Das medizinische Bildgebungsgerät weist zudem einen Bildprojektor auf, der ein Bild auf die Leinwandeinheit für einen Patienten projiziert.

EP 3 581 109 A1 offenbart eine medizinische Vorrichtung mit einem Kamerasystem zur Abbildung eines Teils eines Subjekts umfasst, das auf einem Subjektträger ruht, einem Anzeigesystem zum Rendern einer Positionsrückmeldeanzeige, wobei das Anzeigesystem derart konfiguriert ist, dass die Positionsrückmeldeanzeige für das Subjekt sichtbar ist, wenn das Subjekt auf dem Subjektträger ruht.

EP 3 660 530 A1 beschreibt ein Verfahren zur medizinischen Bildgebung, bei dem fMRT-Daten einer interessierenden Region eines Probanden in einem aktiven Zustand und während einer Gehirnaktivitätsanalysemodus empfangen werden, aus den fMRT-Daten unter Verwendung eines vordefinierten Modells von fMRT-Datenvariationen ein transversale T2*-Karte generiert wird, die generierte T2*-Karte mit einer Referenz-T2*-Karte verglichen wird und eine von einem Blutsauerstoffgehalt abhängige Reaktion der interessierenden Region während des aktuellen aktiven Zeitfensters unter Verwendung der Ergebnisse des Vergleichs geschätzt wird.

Des Weiteren wird in KIM DO YOON ET AL: "Stretchable and reflective displays: materials, technologies and strategies", NANO CONVERGENCE, [Online], Bd. 6, Nr. 1, 20. Juni 2019 (2019-06-20), DOI: 10.1186/s40580-019-0190-5, dass reflektive Displays dehnbar sind. Dabei werden die reflektiven Displays je nach Reaktion auf eine Dehnung in unterschiedliche Typen unterteilt.

US 2006 079 763 A1 offenbart eine Vorrichtung zum Anregen eines Patienten, wobei die Vorrichtung einen Aufbau umfasst, die an eine Patientenliege montiert wird und an die ein Bildschirm angeordnet ist.

JP 6 656881 B2 beschreibt eine Magnetresonanzvorrichtung mit einem von einer Scannereinheit zumindest teilweise umgebenen Patientenaufnahmebereich und einer innerhalb dessen angeordneten Patientendarstellungseinheit zur optischen Ausgabe von Informationen an einen Patienten, die ein reflektives Display umfasst, worauf dem Patienten während der Untersuchung stehende oder bewegte Bilder angezeigt werden können.

Der vorliegenden Erfindung liegt insbesondere die Aufgabe zugrunde, eine einfache und platzsparende Integration einer visuellen Kommunikationseinheit für den Patienten während einer Magnetresonanzvorrichtung zur Verfügung zu stellen. Die Aufgabe wird durch die Merkmale des unabhängigen Anspruchs gelöst. Vorteilhafte Ausgestaltungen sind in den Unteransprüchen beschrieben.

Die Erfindung geht aus von einer Magnetresonanzvorrichtung mit einer Scannereinheit, einem von der Scannereinheit zumindest teilweise umgebenen Patientenaufnahmebereich und einer innerhalb des Patientenaufnahmebereichs angeordneten Patientendarstellungseinheit, wobei die Patientendarstellungseinheit ein reflektives Display umfasst.

Die Magnetresonanzvorrichtung umfasst eine medizinische und/oder diagnostische Magnetresonanzvorrichtung, die zu einem Erfassen von medizinischen und/oder diagnostischen Bilddaten, insbesondere medizinischen und/oder diagnostischen Magnetresonanzbilddaten, eines Patienten ausgelegt und/oder ausgebildet ist. Die Magnetresonanzvorrichtung umfasst hierzu die Scannereinheit. Die Scannereinheit der Magnetresonanzvorrichtung umfasst eine Detektoreinheit, insbesondere eine Magneteinheit, zur Erfassung der medizinischen und/oder diagnostischen Bilddaten. Vorteilhafterweise umfasst hierbei die Scannereinheit, insbesondere die Magneteinheit, einen Grundmagneten, eine Gradientenspuleneinheit und eine Hochfrequenzantenneneinheit. Die Hochfrequenzantenneneinheit ist fest innerhalb der Scannereinheit angeordnet und zur Aussendung eine Anregungspulses ausgelegt und/oder ausgebildet. Zur Erfassung der Magnetresonanzsignale weist die Magnetresonanzvorrichtung lokale Hochfrequenzspulen auf, die um den zu untersuchenden Bereich des Patienten angeordnet werden.

Der Grundmagnet der Scannereinheit ist zur Erzeugung eines homogenen Grundmagnetfelds mit einer definierten und/oder bestimmten Magnetfeldstärke, wie beispielsweise mit einer definierten und/oder bestimmten Magnetfeldstärke von 3 T oder 1,5 T usw., ausgebildet. Insbesondere ist der Grundmagnet zur Erzeugung eines starken, konstanten und homogenen Grundmagnetfelds ausgebildet. Das homogene Grundmagnetfeld ist bevorzugt innerhalb des Patientenaufnahmebereichs der Magnetresonanzvorrichtung angeordnet und/oder vorzufinden. Die Gradientenspuleneinheit ist zu einer Erzeugung von Magnetfeldgradienten, die für eine Ortskodierung während einer Bildgebung verwendet werden, ausgebildet.

Der Patientenaufnahmebereich ist zu einer Aufnahme des des zu untersuchenden Bereichs des Patienten, für eine medizinische Magnetresonanzuntersuchung ausgelegt und/oder ausgebildet. Beispielsweise ist hierzu der Patientenaufnahmebereich zylinderförmig ausgebildet und/oder zylinderförmig von der Scannereinheit umgeben. Hierzu weist die Scannereinheit eine den Patientenaufnahmebereich zumindest teilweise umgebenden Umhausung der Gehäuseeinheit auf. Die den Patientenaufnahmebereich umgebende Umhausung kann hierbei auch einteilig und/oder einstückig mit der dem Patientenaufnahmebereich zugewandte Seite der Hochfrequenzantenneneinheit der Scannereinheit ausgebildet sein oder auch separat zur Hochfrequenzantenneneinheit der Scannereinheit ausgebildet sein.

Innerhalb des Patientenaufnahmebereichs ist bevorzugt ein Field of View (FOV) und/oder ein Isozentrum der Magnetresonanzvorrichtung angeordnet. Das FOV umfasst bevorzugt einen Erfassungsbereich der Magnetresonanzvorrichtung, innerhalb dessen die Bedingungen für eine Erfassung von medizinischen Bilddaten, insbesondere Magnetresonanzbilddaten, vorliegen, wie beispielsweise ein homogenes Grundmagnetfeld. Das Isozentrum der Magnetresonanzvorrichtung umfasst bevorzugt den Bereich und/oder Punkt innerhalb der Magnetresonanzvorrichtung, der die optimalen und/oder idealen Bedingungen für die Erfassung von medizinischen Bilddaten, insbesondere Magnetresonanzbilddaten, aufweist. Insbesondere umfasst das Isozentrum den homogensten Magnetfeldbereich innerhalb der Magnetresonanzvorrichtung.

Die Patientenlagerungsvorrichtung ist zu einer Positionierung und/oder Lagerung des Patienten für eine Magnetresonanzuntersuchung ausgebildet. Die Patientenlagerungsvorrichtung kann dabei einen Patiententisch umfassen, der in den Patientenaufnahmebereich einfahrbar ausgebildet ist. Für eine Magnetresonanzuntersuchung wird der Patient derart auf dem Patiententisch positioniert, dass der zu untersuchende Bereich nach einem Positionieren des Patiententischs innerhalb des Patientenaufnahmebereichs innerhalb des Isozentrums des Patientenaufnahmebereichs angeordnet und/oder positioniert ist.

Für eine Kommunikation und/oder eines Informationsaustauschs des Patienten mit dem medizinischen Bedienpersonal während einer Magnetresonanzuntersuchung weist die Magnetresonanzvorrichtung eine Kommunikationseinheit auf. Die Kommunikationseinheit weist auf Benutzerseite bevorzugt ein Kommunikationselement, wie beispielsweise eine Kommunikationskonsole zur Eingabe und/oder Ausgabe von Kommunikationsdaten, wie beispielsweise Informationen. Des Weiteren weist die Kommunikationseinheit auf Patientenseite ebenfalls zumindest ein Kommunikationselement auf. Insbesondere weist die Kommunikationseinheit ein visuelles Kommunikationselement auf, das als Patientendarstellungseinheit ausgebildet ist.

Die Patientendarstellungseinheit weist eine Darstellungsfläche, insbesondere ein Patientendisplay, auf, das innerhalb des Patientenaufnahmebereichs angeordnet ist. Die Patientendarstellungseinheit, insbesondere das Patientendisplay, ist zur einer visuellen und/oder optischen Ausgabe von Informationen und/oder Anweisungen während der Magnetresonanzuntersuchung an den Patienten ausgebildet. Die Patientendarstellungseinheit, insbesondere das Patientendisplay, weist dabei ein reflektives Display auf. Das reflektive Display umfasst bevorzugt eine passive Anzeige und/oder eine nichtleuchtende Anzeige von Inhalten und/oder Informationen auf dem Display und/oder eine Darstellungsoberfläche. Bevorzugt umfasst das reflektive Display ein E-Paper-Display. Reflektive Displays und/oder E-Paper-Displays reflektieren Licht wie normales Papier. Häufig sind dabei reflektive Displays und/oder E-Paper-Displays derart aufgebaut, dass ein Abstand von bildgebenden Elementen zur Oberfläche, insbesondere einer Ausgabeoberfläche und/oder einer Darstellungsoberfläche, geringer ist als bei herkömmlichen Displays, wie beispielsweise einem LC-Display.

Die Erfindung weist den Vorteil auf, dass ein angezeigter Bildinhalt aufgrund eines geringen Abstands zwischen den bildgebenden Elementen und der Anzeigeoberfläche und/oder Darstellungsoberfläche aus allen Blickwinkeln auf die Anzeigeoberfläche und/oder Darstellungsoberfläche gleich aussieht. Zudem können mittels reflektiver Displays und/oder E-Paper-Displays statische Anzeigen über einen längeren Zeitraum besonders energiesparend realisiert werden, da bei diesen Displays nur zu einem Ändern eines Bildinhalts, beispielsweise einen Seitenwechsel, ein Stromfluss erforderlich ist. Die statische Anzeige kann zudem flimmerfrei betrieben werden. Ein weiterer Vorteil ist, dass eine Anzeige auf dem reflektiven Display und/oder dem E-Paper-Display sowohl in einer dunklen Umgebung als auch in einer hellen Umgebung gut für einen Patienten ablesbar und/oder erkennbar ist. Zudem können reflektive Displays und/oder E-Paper-Displays sehr dünn und leicht ausgebildet werden, so dass eine platzsparende Anordnung innerhalb des Patientenaufnahmebereichs erreicht werden kann und ein dem Patienten zur Verfügung stehender Raum und/oder Platz innerhalb des Patientenaufnahmebereichs im Wesentlichen kaum eingeschränkt wird.

In einer vorteilhaften Weiterbildung der erfindungsgemäßen Magnetresonanzvorrichtung kann es vorgesehen sein, dass der Patientenaufnahmebereich eine den Patientenaufnahmebereich umgebenden Umhausung mit einer Innenwand aufweist und das reflektive Display an der den Patientenaufnahmebereich nach oben umgebenden Innenwand angeordnet ist. Bevorzugt ist das reflektive Display und/oder das E-Paper-Display an einer einem Lagerungsbereich, der zu einer Lagerung eines Kopfes des Patienten ausgelegt und/oder ausgebildet ist, gegenüberliegenden Innenwand der den Patientenaufnahmebereich umgebenden Umhausung angeordnet. Hierdurch kann eine vorteilhafte Sichtbarkeit des reflektiven Displays und/oder des E-Paper-Displays für einen Patienten während einer Magnetresonanzuntersuchung und/oder während eines Aufenthalts innerhalb des Patientenaufnahmebereichs erreicht werden.

In einer vorteilhaften Weiterbildung der erfindungsgemäßen Magnetresonanzvorrichtung kann es vorgesehen sein, dass das reflektive Display eine Form aufweist und die Form des reflektiven Display an eine Oberflächenform der Innenwand der den Patientenaufnahmebereich umgebenden Umhausung angepasst ist. Bevorzugt ist hierbei das reflektive Display und/oder das E-Paper-Display biegbar ausgebildet und kann damit besonders einfach an die Oberflächenform der Innenwand der den Patientenaufnahmebereich umgebenden Umhausung angepasst werden. Derart kann eine besonders platzsparende Anordnung der Patientendarstellungseinheit, insbesondere des reflektive Displays und/oder des E-Paper-Displays, innerhalb des Patientenaufnahmebereichs erreicht werden. Insbesondere kann derart eine passgenaue Anordnung des reflektive Displays und/oder des E-Paper-Displays an die Oberflächenform der Innenwand erreicht werden.

In einer vorteilhaften Weiterbildung der erfindungsgemäßen Magnetresonanzvorrichtung kann es vorgesehen sein, dass die Magnetresonanzvorrichtung eine Datenübertragungseinheit aufweist, die zu einer Datenübertragung an das reflektive Display ausgebildet ist. Die Datenübertragungseinheit kann dabei eine kabelgebundenen Datenübertragung und/oder eine Datenübertragung mittels Lichtwellenleiter und/oder eine Datenübertragung mittels eines Standard-Funkprotokolls, wie beispielsweise mittels Bluetooth, und/oder eine Datenübertragung mittels optischer Datenübertragungstechniken, wie beispielsweise mittels Infrarot-Datenübertragung, umfassen. Zudem sind weitere, dem Fachmann als sinnvoll erscheinende Übertragungstechniken jederzeit möglich.

Erfindungsgemäß weist die Magnetresonanzvorrichtung eine Displaysteuerungseinheit auf, wobei mittels der Displaysteuerungseinheit eine Datenübertragung an das reflektive Display und/oder eine Signalverarbeitung durch das reflektive Display während einer Erfassung von Magnetresonanzdaten deaktiviert wird.

Die Displaysteuerungseinheit umfasst zumindest ein Rechenmodul und/oder einen Prozessor, wobei die Displaysteuerungseinheit zu einer Steuerung der Patientendarstellungseinheit, insbesondere des reflektiven Displays und/oder des E-Paper-Displays, ausgebildet ist. So ist insbesondere die Displaysteuerungseinheit dazu ausgebildet, zur Steuerung der Patientendarstellungseinheit, insbesondere des reflektiven Displays und/oder des E-Paper-Displays, computerlesbare Instruktionen auszuführen. Insbesondere umfasst die Displaysteuerungseinheit eine Speichereinheit, wobei auf der Speichereinheit computerlesbare Informationen gespeichert sind, wobei die Displaysteuerungseinheit dazu ausgebildet ist, die computerlesbaren Informationen von der Speichereinheit zu laden und die computerlesbaren Informationen auszuführen. Die Komponenten der Displaysteuerungseinheit können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützten Hardwarekomponenten, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden. Selbstverständlich ist es auch denkbar, dass mehrere der genannten Komponenten in Form einer einzelnen Softwarekomponente bzw. softwareunterstützter Hardwarekomponente zusammengefasst realisiert sind.

Die Erfassung von Magnetresonanzdaten erfolgt bevorzugt in Magnetresonanzdaten-Erfassungszeitfenstern. Während dieser Magnetresonanzdaten-Erfassungszeitfenster ist die Datenübertragung an das reflektive Display und/oder eine Signalverarbeitung durch das reflektive Display verhindert und/oder deaktiviert. Diese Ausgestaltung der Erfindung weist den Vorteil auf, dass eine unerwünschte Störung einer Magnetresonanzdatenerfassung vorteilhaft verhindert wird und damit auch Bildartefakte in den erfassten Magnetresonanzbilddaten verhindert werden können.

In einer vorteilhaften Weiterbildung der erfindungsgemäßen Magnetresonanzvorrichtung kann es vorgesehen sein, dass während der Erfassung von Magnetresonanzdaten eine statische Darstellung von Bildinformationen und/oder Anweisungen durch das reflektive Display angezeigt wird. Aufgrund der statischen und/oder dauerhaften Darstellung von Bildinformationen und/oder Anweisungen während der Erfassung von Magnetresonanzdaten, insbesondere während der Magnetresonanzdaten-Erfassungszeitfenster, kann der Patient weiterhin Informationen erhalten. Diese statische und/oder dauerhafte Anzeige während der Magnetresonanzdaten-Erfassungszeitfenster kann zu einer Beruhigung des Patienten beitragen und derart auch zu einer hohen Qualität der erfassten Bilddaten beitragen.

Weitere Vorteile, Merkmale und Einzelheiten der Erfindung ergeben sich aus dem im Folgenden beschriebenen Ausführungsbeispiel sowie anhand der Zeichnungen.

Es zeigen:
- Fig. 1: eine erfindungsgemäße Magnetresonanzvorrichtung mit einer Patientendarstellungseinheit in einer schematischen Darstellung und
- Fig. 2: eine Querschnitt durch den Patientenaufnahmebereich mit der Patientendarstellungseinheit.

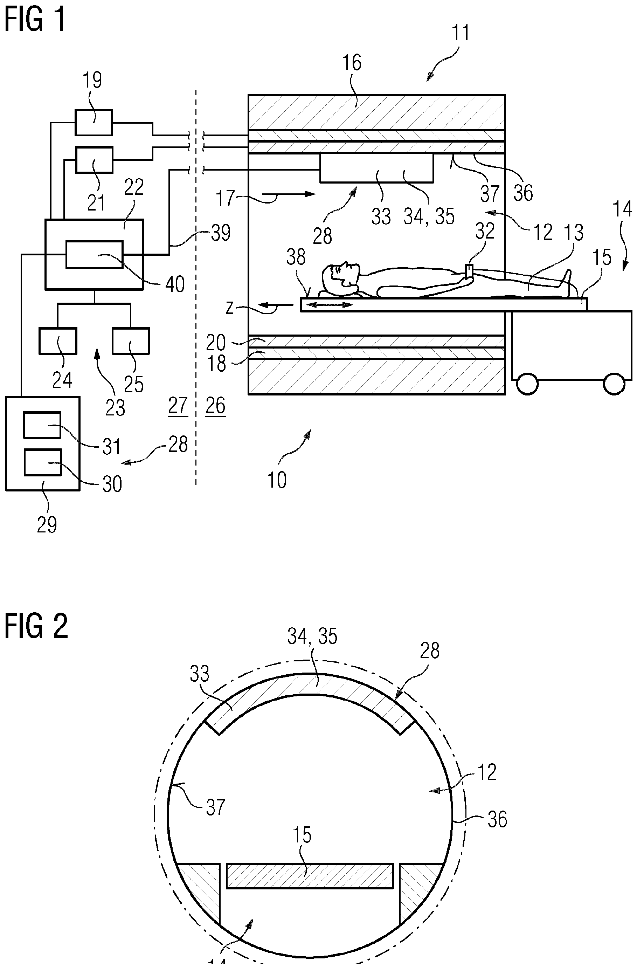

In Fig. 1 ist eine Magnetresonanzvorrichtung 10 schematisch darstellt. Die Magnetresonanzvorrichtung 10 umfasst eine von einer Magneteinheit gebildeten Scannereinheit 11. Zudem weist die Magnetresonanzvorrichtung 10 einen Patientenaufnahmebereich 12 auf zu einer Aufnahme eines Patienten 13. Der Patientenaufnahmebereich 12 im vorliegenden Ausführungsbeispiel ist zylinderförmig ausgebildet und in einer Umfangsrichtung von der Scannereinheit 11, insbesondere von der Magneteinheit, zylinderförmig umgeben. Grundsätzlich ist jedoch eine davon abweichende Ausbildung des Patientenaufnahmebereichs 12 jederzeit denkbar. Der Patient 13 kann mittels einer Patientenlagerungsvorrichtung 14 der Magnetresonanzvorrichtung 10 in den Patientenaufnahmebereich 12 geschoben und/oder gefahren werden. Die Patientenlagerungsvorrichtung 14 weist hierzu einen innerhalb des Patientenaufnahmebereichs 12 bewegbar ausgestalteten Patiententisch 15 auf. Insbesondere ist hierbei der Patiententisch 15 in Richtung einer Längserstreckung des Patientenaufnahmebereichs 12 und/oder in z-Richtung bewegbar gelagert.

Die Scannereinheit 11, insbesondere die Magneteinheit, umfasst einen supraleitenden Grundmagneten 16 zu einem Erzeugen eines starken und insbesondere konstanten Grundmagnetfelds 17. Weiterhin weist die Scannereinheit 11, insbesondere die Magneteinheit, eine Gradientenspuleneinheit 18 zu einer Erzeugung von Magnetfeldgradienten auf, die für eine Ortskodierung während einer Bildgebung verwendet werden. Die Gradientenspuleneinheit 18 wird mittels einer Gradientensteuereinheit 19 der Magnetresonanzvorrichtung 10 gesteuert. Die Scannereinheit 11, insbesondere die Magneteinheit, umfasst weiterhin eine Hochfrequenzantenneneinheit 20 zu einer Anregung einer Polarisation, die sich in dem von dem Grundmagneten 16 erzeugten Grundmagnetfeld 17 einstellt. Die Hochfrequenzantenneneinheit 20 wird von einer Hochfrequenzantennensteuereinheit 21 der Magnetresonanzvorrichtung 10 gesteuert und strahlt hochfrequente Magnetresonanzsequenzen in den Patientenaufnahmebereich 12 der Magnetresonanzvorrichtung 10 ein.

Zu einer Steuerung des Grundmagneten 16, der Gradientensteuereinheit 19 und zur Steuerung der Hochfrequenzantennensteuereinheit 21 weist die Magnetresonanzvorrichtung 10 eine Systemsteuereinheit 22 auf. Die Systemsteuereinheit 22 steuert zentral die Magnetresonanzvorrichtung, wie beispielsweise das Durchführen einer vorbestimmten bildgebenden Gradientenechosequenz. Zudem umfasst die Systemsteuereinheit 22 eine nicht näher dargestellte Auswerteeinheit zu einer Auswertung von medizinischen Bilddaten, die während der Magnetresonanzuntersuchung erfasst werden.

Des Weiteren umfasst die Magnetresonanzvorrichtung 10 eine Benutzerschnittstelle 23, die mit der Systemsteuereinheit 22 verbunden ist. Steuerinformationen wie beispielsweise Bildgebungsparameter, sowie rekonstruierte Magnetresonanzbilder können auf einer Anzeigeeinheit 24, beispielsweise auf zumindest einem Monitor, der Benutzerschnittstelle 23 für ein medizinisches Bedienpersonal angezeigt werden. Weiterhin weist die Benutzerschnittstelle 23 eine Eingabeeinheit 25 auf, mittels der Informationen und/oder Parameter während eines Messvorgangs von dem medizinischen Bedienpersonal eingegeben werden können.

Die Scannereinheit 11 der Magnetresonanzvorrichtung 10 ist zusammen mit der Patientenlagerungsvorrichtung 14 innerhalb eines Untersuchungsraums 26 angeordnet. Die Systemsteuereinheit 22 dagegen ist zusammen mit der Benutzerschnittstelle 23 innerhalb eines Kontrollraums 27 angeordnet. Der Kontrollraum 27 ist getrennt von dem Untersuchungsraum 26 ausgebildet. Insbesondere ist der Untersuchungsraum 26 hinsichtlich einer Hochfrequenzstrahlung von dem Kontrollraum 27 abgeschirmt. Während einer Magnetresonanzuntersuchung befindet sich der Patient 13 innerhalb des Untersuchungsraums 26, dagegen befindet sich das medizinische Bedienpersonal zumeist innerhalb des Kontrollraums 27.

Für eine Kommunikation und/oder eines Informationsaustauschs des Patienten 13 mit dem medizinischen Bedienpersonal während einer Magnetresonanzuntersuchung weist die Magnetresonanzvorrichtung 10 eine Kommunikationseinheit 28 auf (Fig. 1). Die Kommunikationseinheit 28 weist auf Bedienerseite eine als Bedienerkonsole 29 ausgebildetes Kommunikationselement auf. Das Kommunikationselement, insbesondere die Bedienerkonsole 28, ist bevorzugt innerhalb des Kontrollraums 27 angeordnet. Die Bedienerkonsole 29 weist ein Eingabeelement 30 und ein Ausgabeelement 31 auf. Das Eingabeelement 30 und/oder das Ausgabeelement 31 kann dabei als akustisches und/oder visuelles Eingabeelement 30 und/oder Ausgabeelement 31 ausgebildet sein.

Des Weiteren weist die Kommunikationseinheit 28 auf Patientenseite ein erstes Kommunikationselement auf, das als Eingabeelement 32 ausgebildet ist. Mittels des Eingabeelements 32 kann der Patient 13 dem Bediener, insbesondere dem medizinischen Bedienpersonal, ein Befinden, wie beispielsweise ein Unwohlsein, während der Magnetresonanzuntersuchung mitteilen. Im vorliegenden Ausführungsbeispiel ist das Eingabeelement 32 als Patientenrufball ausgebildet. Grundsätzlich sind jedoch weitere, dem Fachmann als sinnvoll erscheinende Eingabeelemente 32, wie beispielsweise ein Mikrofon usw. in einer weiteren Ausbildung der Kommunikationseinheit 28 möglich. Die Kommunikationseinheit 28 weist auf Patientenseite des Weiteren ein zweites Kommunikationselement auf, das als Ausgabeelement 33 ausgebildet ist. Das Ausgabeelement 33 ist als Patientendarstellungseinheit 34 ausgebildet. Die Patientendarstellungseinheit 34 umfasst ein reflektives Display 35, das als E-Paper-Display ausgebildet ist.

Die Patientendarstellungseinheit 34, insbesondere das reflektives Display 35 und/oder das E-Paper-Display, ist innerhalb des Patientenaufnahmebereichs 12 angeordnet. Der Patientenaufnahmebereich 12 umfasst eine die den Patientenaufnahmebereich 12 umgebenden Umhausung 36 mit einer Innenwand 37. Die den Patientenaufnahmebereich 12 umgebenden Umhausung 36 ist im vorliegenden Ausführungsbeispiel einsteilig mit der Hochfrequenzantenneneinheit 20, insbesondere einer dem Patientenaufnahmebereich 12 zugewandten Seite der Hochfrequenzantenneneinheit 20, ausgebildet. In einer alternativen Ausgestaltung der Erfindung kann die den Patientenaufnahmebereich 12 umgebende Umhausung 36 auch ein von der Hochfrequenzantenneneinheit 20 separaten Einheit bilden.

Das reflektive Display 35 und/oder das E-Paper-Display ist an der den Patientenaufnahmebereich 12 nach oben umgebende Innenwand 37 der den Patientenaufnahmebereich 12 umgebenden Umhausung 36 angeordnet. Derart ist das reflektive Display 35 und/oder das E-Paper-Display an einer Seite des Patientenaufnahmebereichs 12 angeordnet, der einer Lagerungsfläche 38 zur Lagerung des Patienten 13, insbesondere des Kopfs des Patienten 13, gegenüberliegend angeordnet ist.

Das reflektive Display 35 und/oder das E-Paper-Display ist besonders dünn und flexibel ausgebildet. Dabei weist das reflektive Display 35 und/oder das E-Paper-Display eine Form auf, die an eine Oberflächenform der Innenwand 37 der den Patientenaufnahmebereich 12 umgebenden Umhausung 36 angepasst ist. Insbesondere ist hierbei das reflektive Display 35 und/oder des E-Paper-Display passgenau an der Oberflächenform der Innenwand 37 angeordnet, wie dies in Fig. 2, einem Querschnitt durch den Patientenaufnahmebereich 12 zu sehen ist.

Die Magnetresonanzvorrichtung 10, insbesondere die Kommunikationseinheit 28, weist des Weiteren eine Datenübertragungseinheit 39 auf, wobei die Datenübertragungseinheit 39 zu einer Datenübertragung von Kommunikationsdaten, insbesondere visuellen Kommunikationsdaten, an das reflektive Display 35 und/oder das E-Paper-Display ausgebildet ist (Fig. 1). Die Datenübertragungseinheit 39 kann dabei zu eine kabelgebundenen Datenübertragung und/oder zu einer Datenübertragung mittels Lichtwellenleiter und/oder einer Datenübertragung mittels eines Standard-Funkprotokolls, wie beispielsweise mittels Bluetooth, und/oder optischer Datenübertragungstechniken, wie beispielsweise mittels Infrarot-Datenübertragung, umfassen. Zudem sind weitere, dem Fachmann als sinnvoll erscheinende Übertragungstechniken jederzeit möglich.

Die Magnetresonanzvorrichtung 10, insbesondere die Kommunikationseinheit 28, weist des Weiteren eine Displaysteuerungseinheit 40 auf. Im vorliegenden Ausführungsbeispiel ist die Displaysteuerungseinheit 40 innerhalb der Systemsteuereinheit 22 integriert. In einer alternativen Ausgestaltung der Displaysteuerungseinheit 40 kann diese auch separat zur Systemsteuereinheit 22 ausgebildet sein.

Mittels der Displaysteuerungseinheit 40 wird eine Kommunikation über das reflektive Display 35 und/oder das E-Paper-Display gesteuert. Dabei ist die Displaysteuerungseinheit 40 dazu ausgebildet, dass mittels der Displaysteuerungseinheit 40 eine Datenübertragung an das reflektive Display 35 und/oder eine Signalverarbeitung durch das reflektive Display 35 während einer Erfassung von Magnetresonanzdaten deaktiviert wird. Die Erfassung von Magnetresonanzdaten erfolgt bevorzugt in Magnetresonanzdaten-Erfassungszeitfenstern. Während dieser Magnetresonanzdaten-Erfassungszeitfenster wird die Datenübertragung an das reflektive Display 35 und/oder eine Signalverarbeitung durch das reflektive Display 35 mittels der Displaysteuerungseinheit 40 verhindert und/oder deaktiviert. Zudem wird während der Erfassung von Magnetresonanzdaten, insbesondere in diesen Magnetresonanzdaten-Erfassungszeitfenstern, mittels des reflektiven Displays 35 und/oder des E-Paper-Displays eine statische und/oder dauerhafte Bildinformation für den Patienten 13 dargestellt werden.

Die dargestellten Magnetresonanzvorrichtung 10 kann selbstverständlich weitere Komponenten umfassen, die Magnetresonanzvorrichtungen 10 gewöhnlich aufweisen. Eine allgemeine Funktionsweise einer Magnetresonanzvorrichtung 10 ist zudem dem Fachmann bekannt, so dass auf eine detaillierte Beschreibung der weiteren Komponenten verzichtet wird.

## Patentansprüche

1. Magnetresonanzvorrichtung mit einer Scannereinheit (11), einem von der Scannereinheit (11) zumindest teilweise umgebenen Patientenaufnahmebereich (12) und einer innerhalb des Patientenaufnahmebereichs (12) angeordneten Patientendarstellungseinheit (34), wobei die Patientendarstellungseinheit (34) zur einer visuellen und/oder optischen Ausgabe von Informationen und/oder Anweisungen während der Magnetresonanzuntersuchung an einen Patienten (13) ausgebildet ist und ein reflektives Display (35) umfasst, wobei das reflektive Display (35) Licht wie normales Papier reflektiert, **gekennzeichnet durch** eine Displaysteuerungseinheit (40), wobei die Displaysteuerungseinheit (40) eingerichtet ist, eine Datenübertragung an das reflektive Display (35) und eine Signalverarbeitung durch das reflektive Display (35) während einer Erfassung von Magnetresonanzdaten zu deaktivieren.

2. Magnetresonanzvorrichtung nach Anspruch 1,
**dadurch gekennzeichnet, dass** das reflektive Display (35) ein E-Paper-Display umfasst.

3. Magnetresonanzvorrichtung nach einem der vorhergehenden Ansprüche,
**dadurch gekennzeichnet, dass** der Patientenaufnahmebereich (12) eine den Patientenaufnahmebereich (12) umgebenden Umhausung (36) mit einer Innenwand (37) aufweist und das reflektive Display (35) an einer Seite des Patientenaufnahmebereichs (12) angeordnet ist, der einer Lagerungsfläche (38) zur Lagerung des Patienten (13), insbesondere des Kopfs des Patienten (13), gegenüberliegend angeordnet ist.

4. Magnetresonanzvorrichtung nach einem der vorhergehenden Ansprüche,
**gekennzeichnet durch** eine Datenübertragungseinheit (39), wobei die Datenübertragungseinheit (39) zu einer Datenübertragung an das reflektive Display (35) ausgebildet ist.

5. Magnetresonanzvorrichtung nach einem der vorhergehenden Ansprüche,
**dadurch gekennzeichnet, dass** während der Erfassung von Magnetresonanzdaten eine statische Darstellung von Bildinformationen durch das reflektive Display (35) angezeigt wird.

## Claims

1. Magnetic resonance apparatus with a scanner unit (11), a patient receiving area (12) at least partly surrounded by the scanner unit (11) and a patient display unit (34) arranged within the patient receiving area (12), wherein the patient display unit (34) is embodied for a visual and/or optical output of information and/or instructions to a patient (13) during the magnetic resonance examination and comprises a reflective display (35), wherein the reflective display (35) reflects light like normal paper,
**characterised by** a display control unit (40), wherein the display control unit (40) is designed to deactivate a data transmission to the reflective display (35) and a signal processing by the reflective display (35) during an acquisition of magnetic resonance data.

2. Magnetic resonance apparatus according to claim 1, **characterised in that** the reflective display (35) comprises an E-paper display.

3. Magnetic resonance apparatus according to one of the preceding claims,
**characterised in that** the patient receiving area (12) has a housing (36) surrounding the patient receiving area (12) with an inner wall (37) and the reflective display (35) is arranged on a side of the patient receiving area (12), which is arranged opposite a support surface (38) for supporting the patient (13), in particular the head of the patient (13).

4. Magnetic resonance apparatus according to one of the preceding claims,
**characterised by** a data transmission unit (39), wherein the data transmission unit (39) is embodied for a data transmission to the reflective display (35).

5. Magnetic resonance apparatus according to one of the preceding claims,
**characterised in that** a static display of image information by the reflective display (35) is shown during the acquisition of magnetic resonance data.

## Revendications

1. Installation de résonance magnétique comprenant une unité (11) de scanner, une partie (12) de réception d'un patient entourée au moins en partie de l'unité (11) de scanner et une unité (34) de représentation d'un patient disposée dans la partie (12) de réception d'un patient, dans laquelle l'unité (34) de représentation d'un patient est constituée pour une sortie visuelle et/ou optique d'informations et/ou d'instructions pendant l'examen par résonance magnétique d'un patient (13) et comprend un écran (35) réfléchissant, dans laquelle l'écran (35) réfléchissant réfléchit la lumière comme du papier normal,
**caractérisée par** une unité (40) de commande d'écran, dans laquelle l'unité (40) de commande d'écran est agencée pour désactiver pendant une saisie de données de résonance magnétique, un transfert de données à l'écran (35) réfléchissant et un traitement de signal par l'écran (35) réfléchissant.

2. Installation de résonance magnétique suivant la revendication 1,
**caractérisée en ce que** l'écran (35) réfléchissant comprend un écran à papier E.

3. Installation de résonance magnétique suivant l'une des revendications précédentes,
**caractérisée en ce que** la partie (12) de réception d'un patient a une enveloppe (36), entourant la partie (12) de réception d'un patient, ayant une paroi (37) intérieure et l'écran (35) réfléchissant est disposé d'un côté de la partie (12) de réception d'un patient, qui est disposée en regard d'une surface (38) de couchette pour coucher le patient (13), en particulier de la tête du patient (13).

4. Installation de résonance magnétique suivant l'une des revendications précédentes,
**caractérisée par** une unité (39) de transmission de données, dans laquelle l'unité (39) de transfert de données est constituée pour un transfert de données à l'écran (35) réfléchissant.

5. Installation de résonance magnétique suivant l'une des revendications précédentes,
**caractérisée en ce que**, pendant la saisie de données de résonance magnétique, une représentation statique d'informations d'image est affichée par l'écran (35) réfléchissant.
